# EUROPEAN PATENT SPECIFICATION

(11) **EP 0 928 340 B1**
(45) Date of publication and mention of the grant of the patent: **10.06.2009**
(21) Application number: 97949632.0
(22) Date of filing: 20.11.1997
(51) Int. Cl.: C12Q 1/02, C12Q 1/00, G01N 33/567, G01N 33/574, G01N 33/53, C12P 21/06, A61K 38/00, A61K 38/04, A61K 35/14, A01N 37/18

(54) **INVASION-INHIBITORS FOR USE IN WOUND HEALING AND CANCER**
INVASIONSINHIBITOREN ZUR VERWENDUNG IN DER WUNDHEILUNG UND KREBSBEHANDLUNG
AGENTS INHIBITEURS D'INVASION S'UTILISANT DANS LA GUERISON DE PLAIES ET LE TRAITEMENT DU CANCER

(30) Priority: 21.11.1996 US 754322; 20.08.1997 US 915189; 18.11.1997 US 972760
(43) Date of publication of application: 14.07.1999
(73) Proprietor: THE REGENTS OF THE UNIVERSITY OF MICHIGAN, Ann Arbor, Michigan 48109-1280 (US)
(72) Inventor: LIVANT, Donna, L., Ann Arbor, MI 48105 (US)
(74) Representative: Glawe, Delfs, Moll
(86) International application number: PCT/US1997/021674
(87) International publication number: WO 1998/022617

(56) References cited:
- WO-A-92/09200
- JP-A- 6 298 797
- US-B- 5 492 890
- DATABASE WPI Section Ch, Week 199502 Derwent Publications Ltd., London, GB; Class B04, AN 1995-011836 XP002286593 & JP 06 298797 A (FUJI PHOTO FILM CO LTD) 25 October 1994 (1994-10-25)
- AKIYAMA S K: "Integrins in cell adhesion and signaling." HUMAN CELL : OFFICIAL JOURNAL OF HUMAN CELL RESEARCH SOCIETY, (1996 SEP) 9 (3) 181-6. REF: 51 JOURNAL CODE: 8912329. ISSN: 0914-7470., September 1996 (1996-09), XP002950878
- CANCER AND METASTASIS REVIEW, 1995, Vol. 14, No. 3, AKIYAMA S. et al., "Fibronectin and Integrins in Invasion and Metastasis", XP000952779

### SEQUENCE LISTING

(1) GENERAL INFORMATION:
   (i) APPLICANT: Livant, Donna L.
   (ii) TITLE OF INVENTION: Invasion-Inducing Agents and Invasion-Inhibitors for Use in Wound Healing and Cancer
   (iii) NUMBER OF SEQUENCES: 6
   (iv) CORRESPONDENCE ADDRESS:
      (A) ADDRESSEE: Medlen & Carroll, LLP
      (B) STREET: 220 Montgomery Street, Suite 2200
      (C) CITY: San Francisco
      (D) STATE: California
      (E) COUNTRY: United States of America
      (F) ZIP: 94104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Carroll, Peter G.
      (B) REGISTRATION NUMBER: 32,827
      (C) REFERENCE/DOCKET NUMBER: UM-03024
   (ix) TELECOMMUNICATION INFORMATION:
      (A) TELEPHONE: (415) 705-8410
      (B) TELEFAX: (415) 397-8338
(2) INFORMATION FOR SEQ ID NO:1:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:1:
   Pro His Ser Arg Asn
   1               5
(2) INFORMATION FOR SEQ ID NO:2:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:2:
   Pro His Ser Cys Asn
   1               5
(2) INFORMATION FOR SEQ ID NO:3:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:3:
   Cys His Ser Arg Asn
   1               5
(2) INFORMATION FOR SEQ ID NO:4:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:4:
   Pro Cys Ser Arg Asn
   1 5
(2) INFORMATION FOR SEQ ID NO:5:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:5:
   Pro His Cys Arg Asn
   1 5
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS: not relevant
      (D) TOPOLOGY: not relevant
   (ii) MOLECULE TYPE: peptide
   (xi) SEQUENCE DESCRIPTION: SEQ ID NO:6:
   Pro His Ser Arg Cys
   1 5

## Description

### FIELD OF THE INVENTION

The present invention relates to invasion-inhibiting peptides for use in the treatment of cancer.

### BACKGROUND

The term "chemotherapy" simply means the treatment of disease with chemical substances. The father of chemotherapy, Paul Ehrlich, imagined the perfect chemotherapeutic as a "magic bullet"; such a compound would kill invading organisms without harming the host. This target specificity is sought in all types of chemotherapeuties, including anticancer agents.

However, specificity has been the major problem with anticancer agents. In the case of anticancer agents, the drug needs to distinguish between host cells that are cancerous and host cells that are not cancerous. The vast bulk of anticancer drugs are indiscriminate at this level. Typically anticancer agents have negative hematological effects (*e*.*g*., cessation of mitosis and disintegration of formed elements in marrow and lymphoid tissues), and immunosuppressive action (*e*.*g*., depressed cell counts), as well as a severe impact on epithelial tissues (*e*.*g*., intestinal mucosa), reproductive tissues (*e*.*g*., impairment of spermatogenesis), and the nervous system. P. Calabresi and B.A. Chabner. In: Goodman and Gilman, The Pharmacological Basis of Therapeutics (Pergamon Press, 8th Edition) (pp. 1209-1216).

Success with chemotherapeutics as anticancer agents has also been hampered by the phenomenon of multiple drug resistance, resistance to a wide range of structurally unrelated cytotoxic anticancer compounds. J.H. Gerlach el al., Cancer Surveys, 5:25-46 (1986). The underlying cause of progressive drug resistance may be due to a small population of drug-resistant cells within the tumor *(e*.*g*., mutant *cells) at* the time of diagnosis. J.H. Goldie and Andrew J. Coldman. Cancer Research, 44:3643-3653 (1984). Treating such a tumor with a single drug first results in a remission, where the tumor shrinks in size as a result of the killing of the predominant drug-sensitive cells. With the drug-sensitive cells gone, the remaining drug-resistant cells continue to multiply and eventually dominate the cell population of the tumor.

WO 92/09200 discloses polypeptides for promoting cell attachment. The polypeptides are derived from human fibronectin and can bind to the GPIIb - IIIa integrin on human platelets.

Akiyama S.K., Hum. Cell 1996, Vol. 9, 181 - 186 reviews the various integrins playing a role in cell adhesion. The peptide motifs PHSRN and RGD are disclosed to act synergistically in the binding of the α₅β₁ integrin to fibronectin.

Finally, the treatment of cancer has been hampered by the fact that there is considerable heterogeneity even within one type of cancer. Some cancers, for example, have the ability to invade tissues and display an aggressive course of growth characterized by metastases. These tumors generally are associated with a poor outcome for the patient. And yet, without a means of identifying such tumors and distinguishing such tumors from non-invasive cancer, the physician is at a loss to change and/or optimize therapy.

What is needed is a specific anticancer approach that is reliable for a wide variety of tumor types, and particularly suitable for invasive tumors. Importantly, the treatment must be effective with minimal host toxicity.

### SUMMARY OF THE INVENTION

The present invention is defined in the claims and relates to invasion-inhibiting peptides for use in the treatment of cancer.

In one embodiment, the present invention provides invasion-inhibiting peptides for treating cancer.

Finally, the present invention contemplates invasion-inhibiting peptides for treating invasive tumors. Specifically, a variety of ariti-invfisive chemotherapeutic agents are contemplated to antagonize the invasion-promoting activity of the PHSRN peptide. In the preferred embodiment, the invasion-inhibitor or "anti-invasive agent" is a peptide with the amino acid sequence PHSCN (Seq. ID No. 2). In another embodiment, the anti-invasive agent is an invasion-inhibiting peptide which has an amino acid sequence comprising PHSXN. where X is an amino acid selected from the group consisting of homo-cysteine and the D-isomer of cysteine.

The present invention also contemplates an anti-invasive agent (*e*.*g*. invasion-inhibiting peptide) comprising the amino acid sequence X₁HSX₂N, wherein X₁ is proline, and X₂ is an amino acid selected from the group consisting of the L-isomer of cysteine, the D-isomer of cysteine, and homo-cysteine. In the preferred embodiment the peptide is PHSCN. where the cysteine is either the L-isomer or D-isomer.

It is further contemplated that the anti-invasive agents named above comprise the named amino acid sequence and additional amino acids added to the amino terminus, the carboxyl terminus, or both the amino and carboxyl termini. In one embodiment, the anti-invasive agent is up to five hundred amino acids in length, and more preferably between four and five hundred amino acids, and still more preferably between six and one hundred amino acids. It is also contemplated that, in some embodiments, the anti-invasive agents named above comprise a peptide with the amino terminus blocked by standard methods to prevent digestion by exopeptidases, for example by acetylation; and the carboxyl, terminus blocked by standard methods to prevent digestion by exopeptidases, for example, by amidation. Further, it is contemplated that, in some embodiments, the anti-invasive agents named above comprise a peptide having one or more L-amino acids replaced by their D-isomers to prevent digestion by endoproteinases.

In this regard, the present invention provides invasion-inhibiting peptides for use in treating cancer comprising: a composition of matter comprising a peptide of claim 1 which inhibits the tumor invasion-promoting activity of the PHSRN sequence of plasma fibronectin. The present invention further contemplates using antagonists of claim 1 before and/or after surgical removal of the primary tumor. In one embodiment, the method comprises PHSRN antagonist of claim 1 as adjunct therapy with additional chemotherapeutics.

While not limited to any mechanism, it is believed that these anti-invasive chemotherapeutic agents antagonize the invasion-promoting activity of the PHSRN sequence (*e*.*g*., of fibronectin) by blocking the binding of this sequence to its receptor on tumor cells. Again while not limited to any mechanism, it is believed that the PHSRN sequence may promote invasion by acting to displace a divalent cation (Mg+.2, Ca+2, or Mn+) in the α5β1 receptor on metastatic tumor cells, and the above named chemotherapeutic anti-invasive agents might act to inhibit this invasion by chelating one or more of these divalent cations.

### DESCRIPTION OF THE FIGURES

Figure 1 schematically shows the one embodiment of the substrate used according to the present invention for testing tumor cells. The spatial relationship of the ectoderm of the *Strongylocentrotus purpuratus* embryo to its extracellular matrix and to blastocoelar structures are shown (s. spicules: h, hyalin layer; e, ectoderm; b. subectodermal basement membrane: bl. blastocoel; g, stomach of the primitive gut; c, coelomic pouches). The esophagus and intestine do not appear on the side of the embryo shown.
Figure 5A is a graph showing the results of inhibiting serum-induced human breast cancer cell invasion of the SU-ECM substrate with varying concentrations of the PHSCN peptides.
Figure 5B is a graph showing the results of inhibiting PHSRN-induced invasion by both human breast cancer cells and normal human mammary epithelial cells of the SU-ECM substrate with varying concentrations of the PHSCN peptide.
Figure 6A is a graph showing the results of inhibiting serum-induced human prostate cancer cell invasion of the SU-ECM substrate with varying concentrations of the PHSCN peptide.
Figure 6B is a graph showing the results of inhibiting PHSRN-induced invasion by both human prostate cancer cells and normal prostate epithelial cells of the SU-ECM substrate with varying concentrations of the PHSCN peptide.
Figure 7A is a graph showing the results of testing serum-induced rat prostate cancer cell invasion of the SU-ECM substrate with and without the PHSCN peptide.
Figure 7B is a graph showing the results of inhibiting PHSRN-induced rat prostate cancer cell invasion of the SU-ECM substrate with varying concentrations of the PHSCN peptide.
Figure 8 is a graph showing the results of inhibiting serum-induced rat prostate cancer cell invasion of the SU-ECM substrate with varying concentrations of the PHS(homo)CN peptide.
Figure 9A is a graph showing the results of testing tumor growth in rats injected with prostate cancer cells, with half of the rats receiving treatment with the PHSCN peptide, initiated in conjunction with the initial injection.
Figure 9B is a graph showing the results of determining the mean number of lung metastases in the two groups of rats described in Figure 9a.
Figure 10A is a graph showing the results of testing tumor growth in rats injected with prostate cancer cells, with half of the rats receiving treatment with the PHSCN peptide, initiated 24 hours after the initial cancer cell injection.
Figure 10B is a graph showing the results of determining the mean number of lung metastases in the two groups of rats described in Figure 10a.
Figure 10C is a graph showing the results of determining the mean mass of intraperitoneal metastatic tissues in the two groups of rats described in Figure 10a.
Figure 11 is a graph showing the results of inhibiting serum-induced human cancer cell invasion with varying concentrations of the PHSCN peptide, as well as PHSCN peptide that has been chemically modified with protecting groups and PHSCN peptide wherein an L-amino acid has been replaced with the D-isomer.
Figure 12 is a graph showing the results of inhibiting serum-induced human cancer cell invasion with varying concentrations of non-peptide compounds in comparison to the PHSCN peptide, as well as PHSCN peptide that has been chemically modified with protecting groups and PHSCN peptide wherein an L-amino acid has been replaced with the D-isomer
Figure 13 is a schematic showing structural relationships between PHSCN and the non-peptide compounds of Figure 12.

### DEFINITIONS

The term "drug" as used herein, refers to any medicinal substance used in humans or other animals. Encompassed within this definition are compound analogs, naturally occurring, synthetic and recombinant pharmaceuticals, hormones, antimicrobials, neurotransmitters, etc.

The term "inducing agent" refers to any compound or molecule which is capable of causing (directly or indirectly) the invasion of cells in a substrate. Thus, invasion inducing agents are defined functionally. This function can be readily assessed by using the invasion substrates and assays of the present invention (described below). "Inducing agents" include, but are not limited to, PHSRN-containing peptides and related peptides (see below).

The term "receptors" refers to structures expressed by cells and which recognize binding molecules (*e*.*g*., ligands).

The term "antagonist" refers to molecules or compounds which inhibit the action of a "native" or "natural" compound (such as fibronectin). Antagonists may or may not be homologous to these natural compounds in respect to conformation, charge or other characteristics. Thus, antagonists may be recognized by the same or different receptors that are recognized by the natural compound. "Antagonists" include, but are not limited to, PHSCN-containing peptides and related peptides (see below).

The term "host cell" or "cell" refers to any cell which is used in any of the screening assays of the present invention. "Host cell" or "cell" also refers to any cell which either naturally expresses particular receptors of interest or is genetically altered so as to produce these normal or mutated receptors. Cells can be transfected with nucleic acid encoding a gene of interest (*i*.*e*. a gene encoding a particular protein, including but not limited to proteins that are therapeutic). The peptides of the present invention are useful to facilitate and enhance the process of introducing nucleic acid into cells.

The term "chemotherapeutic agent" refers to molecules or compounds which inhibit the growth or metastasis of tumors. "Chemotherapeutics" include, but are not limited to, PHSCN-containing peptides and related peptides (see below).

The present invention also contemplates homo-cysteine, which is identified as "hC".

The term "wound" refers broadly to injuries to the skin and subcutaneous tissue initiated in different ways (*e*.*g*., pressure sores from extended bed rest and wounds induced by trauma) and with varying characteristics. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (*e*.*g*., a bony pressure point such as the greater trochanter or the sacrum). The term "partial thickness wound" refers to wounds that encompass Grades I-III; examples of partial thickness wounds include bum wounds, pressure sores, venous stasis ulcers, and diabetic ulcers. The term "deep wound" is meant to include both Grade III and Grade IV wounds. The present invention contemplates treating all wound types, including deep wounds and chronic wounds.

The term "chronic wound" refers to a wound that has not healed within 30 days.

The phrase "positioning the solid support in or on the wound" is intended to mean contacting some part of the wound with the solid support.

The phrases "promote wound healing," "enhance wound healing," and the like refer to either the induction of the formation of granulation tissue of wound contraction and/or the induction of epithelialization (*i*.*e*., the generation of new cells in the epithelium). Wound healing is conveniently measured by decreasing wound area.

The phrase "wound fluid contents" refers to liquid associated with a wound, as well as cells, cell factors, ions, macromolecules and protein material suspended such liquid at the wound site.

The term "subject" refers to both humans and animals.

The terms "enclosure," "compartment," and the like refer broadly to any container capable of confining a solid support within a defined location.

The term "solid support" refers broadly to any support, including, but not limited to, microcarrier beads, gels, Band-Aids™ and dressings.

The term "dressing" refers broadly to any material applied to a wound for protection, absorbance, drainage, etc. Thus, adsorbent and absorbent materials are specifically contemplated as a solid support. Numerous types of dressings are commercially available, including films (*e*.*g*., polyurethane films), hydrocolloids (hydrophilic colloidal particles bound to polyurethane foam), hydrogels (cross-linked polymers containing about at least 60% water), foams (hydrophilic or hydrophobic), calcium alginates (nonwoven composites of fibers from calcium alginate), and cellophane (cellulose with a plasticizer) [Kannon and Garrett, Dermatol. Surg. 21:583-590 (1995); Davies, Burns 10:94 (1983)]. The present invention specifically contemplates the use of dressings impregnated with the wound healing promoting and enhancing compounds of the present invention.

The term "biocompatible" means that there is minimal (*i*.*e*., no significant difference is seen compared to a control), if any, effect on the surroundings. For example, in some embodiments of the present invention, the dressing comprises a biocompatible membrane.

The term "fibronectin-derived peptide" means a peptide that is smaller than the intact fibronectin protein but that has sequence identical (or at least 90% identical) to a portion of the natural fibronectin sequence. For example, the peptide PHSRN has a sequence that exists in a portion of the natural fibronectin; the peptide PHSCN, while not existing as a portion of the natural sequence is, by this definition, a fibronectin-derived peptide. Typically, the peptide will be between four and one hundred amino acids (although larger fragments of fibronectin are possible, including but not limited to fragments wherein additional non-fibronectin amino acid sequences have been added to the peptide). A preferred fibronectin-derived peptide is one lacking the RGD motif of fibronectin. In yet another embodiment, said peptide lacks the motif which binds the α5β1 receptor.

The term "peptide derivative" refers to compound having an imino group (-NH-), and more particularly, a peptide bond. Peptides may be regared as substituted amides. Like the amide group, the peptide bond shows a high degree of resonance stabilization. The C-N single bond in the peptide linkage has typically about 40 percent double-bond character and the C=O double bond about 40 percent single-bond character. Peptide derivatives include (but are not limited to) "peptide analogs" which are herein defined as compounds that can be incorporated into polypeptide chains in place of the corresponding natural amino acids by the natural enzymes (i.e. incorporated by aminoacyl-tRNA synthetases. Examples of such analogues include (but are not limited to) p-fluorophenylalanine (an analog of phenylalanine) and ethionine and norleucine (analogs of methionine).

"Protecting groups" are those groups which prevent undesirable reactions (such as proteolysis) involving unprotected functional groups. In one embodiment, the present invention contemplates that the protecting group is an acyl or an amide. In one embodiment, the acyl is acetate. In another embodiment, the protecting group is a benzyl group. In another embodiment, the protecting group is a benzoyl group. The present invention also contemplates combinations of such protecting groups.

The term "Band-Aid^{™}" is meant to indicate a relatively small adhesive strip comprising an adsorbent pad (such as a gauze pad) for covering minor wounds.

### DESCRIPTION OF THE INVENTION

The present invention is defined in the claims and provides invasion-inhibiting peptides for use in the treatment of cancer.

### I. Diagnosis And Treatment Of Cancer

As a prelude to metastasis, it is believed that cancer cells proteolytically alter basement membranes underlying epithelia or the endothelial linings of blood and lymphatic vessels, invade through the defects created by proteolysis, and enter the circulatory or lymphatic systems to colonize distant sites. During this process, the secretion of proteolytic enzymes is coupled with increased cellular motility and altered adhesion. After their colonization of distant sites, metastasizing tumor cells proliferate to establish metastatic nodules.

As noted above, chemotherapeutic agents are currently employed to reduce the unrestricted growth of cancer cells, either prior to surgical removal of the tumor (neoadjuvant therapy) or after surgery (adjuvant therapy). However, none of these methods has proved curative once metastasis has occurred. Since unrestricted invasive behavior is also a hallmark of metastatic tumor cells, methods for directly inhibiting tumor cell invasion and metastasis are needed.

### A. Assays For Testing Tumor Invasion

Discovering how to inhibit the invasive behavior of tumor cells to intervene in the metastatic cascade first requires the development of assays with which to test tumor cell invasion *in vitro.* Two assay systems are contemplated for use in the method of the present invention to test the tumor cell invasion.

### 1. Fibronectin-Depleted Substrates

In one assay system, the present invention contemplates using fibronectin-depleted substrates. These are substrates that originally contain fibronectin that are treated according to the methods of the present invention (see below) to remove fibronectin. It is not intended that the present invention be limited by the nature of the original substrate; such fibronectin-containing substrates suitable for treatment and depletion include: i) complex substrates containing a variety of extracellular proteins and ii) less complex substrates containing fibronectin along with one or two other proteins (*e*.*g*., collagen, laminin, etc.).

It is also not intended that the present invention be limited by the precise amount of fibronectin remaining after the substrate has been treated. In other words, while the methods of the present invention remove fibronectin, and in some embodiments, remove substantially all fibronectin, it is within the meaning of the term "fibronectin-depleted" substrate that a small amount of fibronectin remain in the substrate.

In one embodiment, the present invention contemplates using an extracellular matrix available commercially. For example, the present invention contemplates treating basement membrane matrices such as ECM GEL, a matrix from mouse sarcoma (commercially available from Sigma, St. Louis, Mo). However, it is not intended that the present invention be limited by the particular fibronectin-containing substrate. For example, other commercially available substrates are contemplated, such as the commonly used substrate Matrigel (available from Becton Dickinson Labware, Catalog #40234); Matrigel can be treated appropriately according to the methods of the present invention so as to render it "fibronectin-depleted" (see below). Untreated Matrigel (and similar substrates) have been used to demonstrate the importance of proteases and motility factors in the invasion and metastasis of many tumors. However, these invasion substrates are not available as serum-free substrates; thus, the regulation of tumor cell invasive behavior by serum components, such as plasma fibronectin, is a complicating factor with untreated Matrigel.

Consequently, the present invention contemplates a fibronectin-free substrate. In this embodiment, Matrigel is treated so that it is substantially fibronectin-free. The preparation of fibronectin-free Matrigel involves "panning" the Matrigel substrate on gelatin as well as "panning" the substrate on anti-fibronectin antibody (anti-human fibronectin IgG is available commercially, such as antibody from Promega Corporation, Madison. Wisconsin).

### 2. Naturally Occurring Fibronectin-Free Substrates

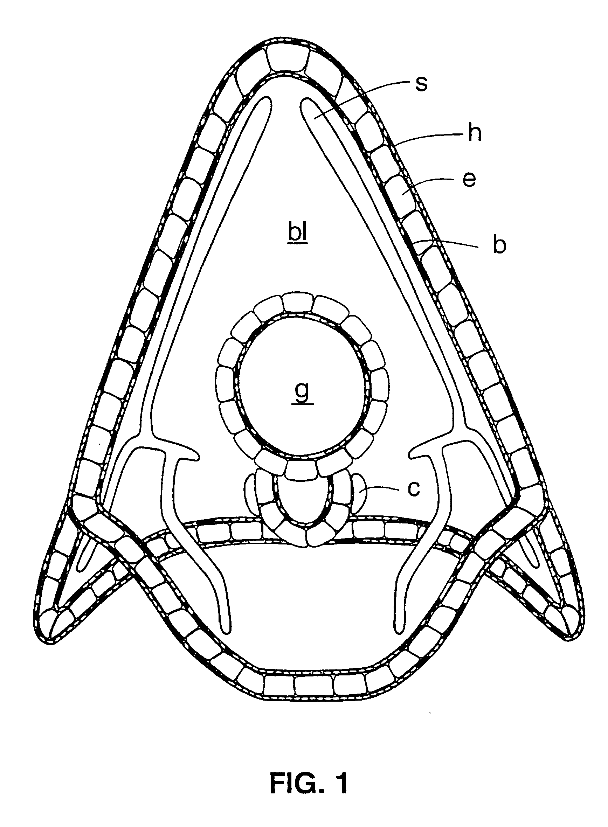

In another embodiment, the present invention contemplates substrates that are naturally free of fibronectin; such a source provides, for example, basement membranes permeable to select types of normally invasive cells, such membranes being naturally serum-free. In one embodiment, the present invention contemplates sea urchins as a source of such membranes. In this regard, the ectoderm of sea urchin embryos is one cell thick, and secretes an underlying basement membrane (see Figure 1) very similar to that of mammals. These embryos contain no circulatory or lymphatic systems; and thus, their basement membranes are serum-free. In embryos, the subectodermal basement membrane functions simultaneously as a migration substrate for several, specific mesenchymal cell types while it functions as an invasion substrate for others. Sea urchin embryo basement membranes (SU-ECM) can be prepared by mild detergent treatment as described in D. Livant et al., Cancer Research 55:5085 (1995) and described in the Experimental section below.

Regardless of which of the two types of substrates are employed, the invasion substrates of the present invention are easy to prepare and give rapid, highly consistent results with a variety of cells, including: a) cell lines from: i) primary and metastatic tumors, and ii) normal epithelial tissues; as well as b) cells from primary tissue samples of both tumors, their surrounding normal tissues, and neonatal melanocytes, fibroblasts, and keratinocytes from circumcised tissue.

In this drug screening assay, candidate drug inhibitors are added to the tissue culture (this can be done individually or in mixtures). Where the inducible tumor cell is found to be inhibited from invading the substrate, a drug inhibitor is indicated (see Examples section below using the PHSCN peptide).

### C. Invasion-Inducing Agents And Antagonists

While an understanding of the mechanisms involved in metastatic cancer is not necessary to the successful practice of the present invention, it is believed that tumor cell invasion of basement membranes occurs at several points in the metastatic cascade: (1) when epithelial tumor cells (such as those of breast and prostate cancers) leave the epithelium and enter the stroma; (2) when tumor cells enter the circulatory or lymphatic systems, and (3) when tumor cells leave the circulatory or lymphatic systems to invade distant sites. Thus, intervention in the *induction* of tumor cell invasiveness by using a PHSRN antagonist, such as the PHSCN peptide, to block tumor cell receptors for this sequence is contemplated as a method for decreasing the rate of metastasis.

One advantage of this strategy is that leukocytes are the only normal cells known to invade tissues routinely to carry out their functions, and relatively few leukocytes are invasive at a given time. Thus, relatively small doses of an anti-invasion antagonist which blocks the binding of PHSRN to its receptor are required. Also, other than some immunodepression, there should be relatively few side effects associated with anti-metastatic treatment using compounds designed to block the induction of invasion. The lack of debilitating side effects expected from anti-invasive therapy means that using it in combination with anti-proliferative agents would be uncomplicated, and that it could be used prior to surgery or even prophylactically to block tumor cell invasion and metastasis.

The IKVAV sequence of laminin, a prevalent insoluble protein of the extracellular matrix, is known to stimulate liver colonization by metastatic human colon cancer cells in athymic mice [*see* Bresalier et al., Cancer Research 55:2476 (1995)]. Since IKVAV, like PHSRN, contains a basic amino acid (K) which, by virtue of its positive charge, might also function to displace a divalent cation from its integrin receptor and stimulate invasion,the present invention contemplates applying the strategy of developing anti-invasion antagonists to the IKVAV sequence of laminin.

**TABLE 1**

| Designation And Origin Of Human Cell Lines And Strains¹ | |
|---|---|
| **Origin** | **Cell Lines or Strains** |
| Colonic carcinoma | SW1116, HCT116, SKCO-1, HT-29, KM12C, KM12SM, KM12L4, SW480 |
| Pancreatic carcinoma | BxPC-3, AsPC-1, Capan-2, MIA PaCa-2, Hs766T |
| Colon adenoma | VaCo 235 |
| Lung carcinoma | A549 |
| Prostate carcinoma | PC-3, DU-145 |
| Breast carcinoma | 009P, 013T, SUM-52 PE |
| Lymphoma | Daudi, Raji |
| Breast epithelium | 006FA |
| Diploid fibroblast | HCS (human corneal stroma), MRC-5 |

| | |
|---|---|
| The SW1116. HT-29, SW480, Raji lymphoblastoid cells, and the pancreatic lines are obtained from the American Type Culture Collection. | |

### 1. Antagonists

A variety of anti-invasive peptides are contemplated to antagonize the invasion-promoting activity of the PHSRN sequence, as defined in the claims.

In the preferred embodiment, the anti-invasive agent is a peptide with the amino acid sequence PHSCN.

In another embodiment, the anti-invasive agent is a peptide which has an amino acid sequence comprising PHSXN, where X is an amino acid selected from the group consisting of homo-cysteine, and the D-isomer of cysteine.

The present invention also contemplates an anti-invasive agent comprising the amino acid sequence X₁HSX₂N, wherein X₁ is proline, and X₂ is an amino acid selected from the group consisting of the L-isomer of cysteine, the D-isomer of cysteine, and homo-cysteine.

It is further contemplated that, in some embodiments the anti-invasive agents named above comprise the named amino acid sequence and additional amino acids added to the amino terminus, the carboxyl terminus, or both the amino and carboxyl termini (in the manner set forth above for the PHSRN containing peptides, *e*.*g*., PHSRNSIT). In one embodiment, the anti-invasive agent is up to five hundred amino acids in length. It is also contemplated that, in some embodiments, the anti-invasive agents named above comprise a peptide with the amino terminus blocked by standard methods to prevent digestion by exopeptidases, for example by acetylation; and the carboxyl terminus blocked by standard methods to prevent digestion by exopeptidases, for example, by amidation.

In this regard, the present invention provides invasion-inhibiting peptides for use in treating cancer comprising: a composition of matter comprising a peptide as defined in claim 1 which inhibits the tumor invasion-promoting activity of a peptide comprising the amino acid sequence PHSRN.

The present invention further contemplates using antagonists as defined in claim 1 before and/or after surgical removal of the primary tumor. In one embodiment, the method comprises the use of a PHSRN antagonist as defined in claim 1 as adjunct therapy with additional chemotherapeutics.

White not limited to any mechanism, it is believed that these anti-invasive chemotherapeutic agents antagonize the invasion-promoting activity of the PHSRN sequence (*e*.*g*., of fibronectin) by blocking the binding of this sequence to its receptor on tumor cells. Again, while not limited to any mechanism, it is believed that the PHSRN sequence may promote invasion by acting to displace a divalent cation (Mg+2, Ca+2. or Mn+) in the α5β1 receptor on metastatic tumor-cells, and the above named chemotherapeutic anti-invasive agents might act to inhibit this invasion by chelating one or more of these divalent cations.

### 2. Designing Mimetics

Compounds mimicking the necessary conformation for recognition and docking to the receptor binding to the peptides of the present invention are contemplated as within the scope of this disclosure. For example, mimetics of PHSRN and PHSRN-antagonists are contemplated. A variety of designs for such mimetics are possible. For example, cyclic PHSRN and PHSCN containing peptides, in which the necessary conformation for binding is stabilized by nonpeptides, are contemplated United States Patent No. 5,192,746 to Lobl, et al., United States Patent No. 5,169,862 to Burke, Jr., et al., United States Patent No. 5,539,085 to Bischoff, et al., United States Patent No. 5,576,423 to Aversa, et al., United States Patent No. 5,051,448 to Shashoua, and United States Patent No. 5,559,103 to Gaeta, et al. describe multiple methods for creating such compounds.

Synthesis of nonpeptide compounds that mimic peptide sequences is also known in the art. Eldred, et al., (J. Med. Chem. 37:3882 (1994)) describe nonpeptide antagonists that mimic the Arg-Gly-Asp sequence. Likewise, Ku, el al., (J. Med. Chem. 38:9 (1995)) give further elucidation of the synthesis of a series of such compounds.

### 4. Administering Chemotherapeutics

It is contemplated that the antagonists of the present invention be administered systemically or locally to inhibit tumor cell invasion in cancer patients with locally advanced or metastatic cancers. They can be administered intravenously, intrathecally, intraperitoneally as well as orally. PHSRN antagonists (e.g., the PHSCN peptide), can be administered alone or in combination with anti-proliferative drugs in a neoadjuvant setting to reduce the metastatic load in the patient prior to surgery; or they can be administered after surgery. Since PHSRN antagonists may depress wound healing (because the PHSRN sequence also elicits fibroblast invasion as described below), it may be necessary to use PHSRN antagonists some time after surgery to remove the tumor.

Since few cells in the body must invade in order to function. PHSRN antagonists administered systemically are not likely to cause the debilitating side effects of cytotoxic chemotherapeutic agents. However, since they suppress invasion, they are likely to cause some immunodepression. Even so, at the appropriate dosage, PSHRN antagonists may be administered prophylactically. In any case, it is contemplated that they may be administered in combination with cytotoxic agents. The simultaneous selection against the two fatal attributes of metastatic cells, unrestricted proliferation and invasion, is contemplated as a very powerful therapeutic strategy.

Where combinations are contemplated, it is not intended that the present disclosure be limited by the particular nature of the combination. The present disclosure contemplates combinations as simple mixtures as well as chemical hybrids. An example of the latter is where the antagonist is covalently linked to a targeting carrier or to an active pharmaceutical. Covalent binding can be accomplished by any one of many commercially available crosslinking compounds.

It is not intended that the present disclosure be limited by the particular nature of the therapeutic preparation. For example, such compositions can be provided together with physiologically tolerable liquid, gel or solid carriers, diluents, adjuvants and excipients.

These therapeutic preparations can be administered to mammals for veterinary use, such as with domestic animals, and clinical use in humans in a manner similar to other therapeutic agents. In general, the dosage required for therapeutic efficacy will vary according to the type of use and mode of administration, as well as the particularized requirements of individual hosts.

Such compositions are typically prepared as liquid solutions or suspensions, or in solid forms. Oral formulations for cancer usually will include such normally employed additives such as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained release formulations, or powders, and typically contain 1 %-95% of active ingredient, preferably 2%-70%.

The compositions are also prepared as injectables, either as liquid solutions or suspensions; solid forms suitable for solution in, or suspension in, liquid prior to injection may also be prepared.

The antagonists of the present invention are often mixed with diluents or excipients which are physiological tolerable and compatible. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH buffering agents.

Additional formulations which are suitable for other modes of administration, such as topical administration, include salves, tinctures, creams, lotions, and, in some cases, suppositories. For salves and creams, traditional binders, carriers and excipients may include, for example, polyalkylene glycols or triglycerides.

### A. Assays For Testing Invasion Potential

### And Screening New Therapeutics

It may be desirable to test the potential of cells for invasion, thereby predicting the ability of a patient to respond to the wound treatment. Similarly, it may be desirable to screen new potential therapeutics for their level of inducing activity. Two assay systems are contemplated for such testing.

### 1. Fibronectin-depleted Substrates

In one assay system, the present disclosure contemplates using fibronectin-depleted substrates. These are substrates that originally contain fibronectin that are treated according to the methods of the present invention (see below) to remove fibronectin. It is not intended that the present disclosure be limited by the nature of the original substrate; such fibronectin-containing substrates suitable for treatment and depletion include i) complex substrates containing a variety of extracellular proteins and ii) less complex substrates containing fibronectin along with one or two other proteins (*e*.*g*. collagen, laminin, etc.).

It is also not intended that the present disclosure be limited by the precise amount of fibronectin remaining after the substrate has been treated. In other words, while the methods of the present disclosure remove fibronectin, and in some embodiments, remove substantially all fibronectin, it is within the meaning of the term "fibronectin-depleted" substrate that a small amount of fibronectin remain in the substrate.

In one embodiment, the present disclosure contemplates using an extracellular matrix available commercially. For example, the present disclosure contemplates treating basement membrane matrices such as ECM GEL, a matrix from mouse sarcoma (commercially available from Sigma. St. Louis, Mo). However, it is not intended that the present disclsure be limited by the particular fibronectin-containing substrate. For example, other commercially available substrates are contemplated, such as the commonly used substrate Matrigel (available from Becton Dickinson Labware. Catalog #40234); Matrigel can be treated appropriately according to the methods of the present invention so as to render it "fibronectin-depleted" (see below).

Consequently, the present disclosure contemplates a fibronectin-free substrate. In this embodiment, Matrigel is treated so that it is substantially fibronectin-free. The preparation of fibronectin-free Matrigel involves "panning" the Matrigel substrate on gelatin as well as "panning" the substrate on anti-fibronectin antibody (anti-human fibronectin IgG is available commercially, such as antibody from Promega Corporation. Madison, Wisconsin).

### 2. Naturally Occurring Fibronectin-free Substrates

In another embodiment, the present disclosure contemplates substrates that are naturally free of fibronectin; such a source provides, for example, basement membranes permeable to select types of normally invasive cells, such membranes being naturally serum-free. In one embodiment, the present disclosure contemplates sea urchins as a source of such membranes. In this regard, the ectoderm of sea urchin embryos is one cell thick, and secretes an underlying basement membrane (see Figure 1) very similar to that of mammals. These embryos contain no circulatory or lymphatic systems: and thus, their basement membranes are serum-free. In embryos, the subectodermal basement membrane functions simultaneously as a migration substrate for several, specific mesenchymal cell types while it functions as an invasion substrate for others.

Sea urchin embryo basement membranes (SU-ECM) can be prepared by mild detergent treatment as described in D. Livant et al., Cancer Research 55:5085 (1995). Briefly, adult *Strongylocentrotus purpuratus* sea urchins can be obtained commercially (*e*.*g*. from Pacific BioMarine), and their embryos cultured to the early pluteus stage in artificial sea water at 15°C. SU-ECM are then prepared from them by treatment with nonionic detergent and strerilized by dilution in the appropriate media.

Cells for the invasion assay are harvested by rinsing in Hanks' balanced salt solution, followed by brief treatment with 0.25% trypsin, 0.02% EDTA, and pelleting and resuspension in the appropriate medium with or without 5% FCS at a density of about 50,000 cells per ml. When appropriate, purified bovine plasma fibronectin (Sigma), purified 120 kDa chymotryptic fragment (Gibco BRL) or PHSRN peptides (synthesized at the Biomedical Research Core Facilities of the University of Michigan) are added to the resuspended cells prior to placement of the cells on SU-ECM. In each well of a plate used for an invasion assay, SU-ECM were placed in 0.5 ml of the appropriate medium, and 0.5ml of the resuspended cells dropped on their exterior surfaces. Invasion assays were incubated 1 to 16 hours prior to assay. If some circumstances, invasion assays were fixed in phosphate-buffered saline (PBS) with 2% formaldehyde for 5 minutes at room temperature, then rinsed into PBS.

Invasion assays are coded and scored blindly by microscopic examination under phase contrast at 200- and 400-fold magnification. Each cell contacting an SU-ECM is scored for its position relative to the exterior or interior surfaces. A cell is judged to have invaded if it is located on an interior surface below the focal plane passing through the upper surface of the SU-ECM. but above the focal plane passing through its lower surface. The minimum viability of the cells in each assay is always ascertained at the time of assay by determining the traction of spread, adherent cells on the bottom of each well scored.

An invasion frequency is defined as the fraction of cells in contact with basement membranes which are located in their interiors at the time of assay. Thus, an invasion frequency of 1 denotes invasion by 100% of the cells in contact with basement membranes. Invasion frequencies are determined multiple times for each cell type assayed. For each type of cell assayed the mean and standard deviation of the invasion frequencies were calculated.

Regardless of which of the two types of substrates are employed, the invasion substrates of the present invention are easy to prepare and give rapid, highly consistent results with a variety of cells, including but not limited to fibroblasts and keratinocytes.

While not limited to any mechanism, it is believed that cells exposed to invasion-inducing agents in this manner are potentially rendered capable of invading the substrate. Again, while not limited to any mechanism, it is believed that the invasion inducing agent comprising the sequence PHSRN binds to the α5β1 receptor on the cell and thereby induces invasion of the substrate.

### EXPERIMENTAL

The following examples serve to illustrate certain preferred embodiments and aspects of the present invention and are not to be construed as limiting the scope thereof.

In the experimental disclosure which follows, the following abbreviations apply: eq (equivalents); µ (micron); M (Molar); µM (micromolar); mM (millimolar); N (Normal); mol (moles); mmol (millimoles); µmol (micromoles); nmol (nanomoles); g (grams); mg (milligrams); µg (micrograms); ng (nanograms); L (liters): ml (milliliters); µl (microliters); cm (centimeters); mm (millimeters); µm (micrometers); nM (nanomolar);°C (degrees Centigrade); mAb (monoclonal antibody); MW (molecular weight); PBS (phophate buffered saline); U (units); d(days).

In some of the examples below, wounds are created in animals. Briefly, in one approach, experimental wounds were created in animals which were pre-anesthetized by inhalation of metofane and intradermal injection of lidocane. The hair on the backs of the animals was clipped and the skin was disinfected with 70% ethanol. A piece of skin was then removed from the disinfected site with a 4 mm punch biopsy.

### EXAMPLE 1

### Production Of Fibronectin-Free Substrates

This example describes a purification approach for removal of plasma fibronectin (and/or cellular fibronectin) from a substrate (Matrigel). In this example, removal was attempted by affinity chromatography over Gelatin-Sepharose (a technique which can be used to remove plasma fibronectin from fetal calf serum).

The Gelatin-Sepharose beads were obtained from Pharmacia (Catalog# 17-0956-01). Two Kontes columns were set up with about 2 mls of Gelatin-Sepharose beads at 4 C to prevent gelling of the Matrigel. The columns were then rinsed with about 10 column volumes of PBS to remove the preservative from the beads. The columns were drained to the top of the beads; then Matrigel was carefully added to the column. Once the Matrigel had entered the column, PBS was added to the top of the column. The Matrigel which was passed over the first column was collected and passed over the second column. The fibronectin-depleted Matrigel collected from the second column was plated on 48-well plates (150 µl/well), sterilized under a UV light for 10 minutes and incubated at 37 C overnight. The Matrigel treated in this manner failed to form a gel at 37 C.

### EXAMPLE 2

### Production Of Fibronectin-Free Substrates

This example describes a purification approach for removal of plasma fibronectin (and/or cellular fibronectin) from a substrate (Matrigel). In this example, removal was attempted by successive panning on gelatin. Eight wells of 24-well plate were coated with a 2% gelatin solution (the gelatin was obtained from Becton Dickinson Labware. Catalog #11868). The wells were filled with the gelatin solution which had been heated to 50 C and incubated for 3 minutes. Then the solution was removed and the wells were allowed to air dry. Following drying, the wells were thoroughly rinsed with ddH2O followed by two rinses with PBS. The plates were again allowed to dry; thereafter they were stored at -20 C until use. Matrigel was thawed on ice and then added to one of the wells of a gelatin-coated plate (between 800 µl and 1 ml of Matrigel was added to a well of a 24-well plate). The plate was placed in a bucket of ice in a 4 C room on an orbital shaker where the Matrigel was incubated in the well for two hours (although overnight incubation can be used). Following the incubation, the Matrigel was moved from the first well to a second well and then incubated for two hours under the same conditions. This process was repeated until the Matrigel had been incubated on all eight wells of the gelatin-coated plate.

Following the depletion of the Matrigel, it was collected in Eppendorf tubes. It was then plated on a 48-well plate 150 µl/well), sterilized under a UV light for 10 minutes and incubated at 37 C overnight. The Matrigel formed as gel and the following day, cells were added to each well.

### EXAMPLE 3

### Production Of Fibronectin-Free Substrates

This example describes a purification approach for removal of plasma fibronectin (and/or cellular fibronectin) from a substrate (Matrigel). In this example, removal was attempted by gelatin panning followed by antibody panning.

*Anti-fibronectin antibody-coated wells*: Wells of a 24-well plate were coated with an anti-fibronectin antibody. A mouse monoclonal antibody to human fibronectin was obtained from Oncogene Science (Catalog #CP13). Each well was incubated with 1 ml of antibody at a concentration of 30 µl/ml for 2 hours at room temperature. Each well was then incubated with a solution of 3% BSA in PBS for 2 hours at room temperature. Following the two incubation periods, the wells were thoroughly washed with PBS and stored at -20 C until use.

*Depleting Matrigel of Fibronectin:* Matrigel was panned over eight gelatin-coated wells (as described above in Example 2) to remove most of the fibronectin and its fragments. Thereafter, the Matrigel was placed in the antibody-coated wells to remove any remaining fragments of fibronectin which contain the cell-binding domain but not the gelatin-binding domain. The Matrigel was incubated in an ice bucket on an orbital shaker at 4 C for 2 hours. Once the Matrigel was depleted, it was collected in Eppendorf tubes. The firbonectin-depleted Matrigel was plated on a 48-well plate (150 µl/well), sterilized under a UV light for 10 minutes and incubated at 37 C overnight. The Matrigel formed a gel and the following day, cells were added to the wells.

### EXAMPLE 4

### Inducing Invasive Behavior Of Tumor Cells

In this example, the role of plasma fibronectin in inducing the invasive behaviors of metastatic breast and prostate cancer cells is demonstrated. Human breast carcinoma cell lines SUM 52 PE and SUM 44 PE were originally cultured from the pleural effusions of patients with metastatic breast cancer; and SUM 102 was cultured from a primary, microinvasive breast carcinoma (Ethier, S.P., Mahack, M.L., Gullick. W.J., Frank, T.S., and Weber, B.L. Differential isolation of normal luminal mammary epithelial cells and breast cancer cells from primary and metastatic sites using selective media. Cancer Res. 53: 627-635). The DU 145 metastatic human prostate cancer cell line was originally cultured from a brain metastasis (Stone, K.R., Mickey, D.D., Wunderli, H., Mickey, G.H., Paulsen. D.F. (1978) Isolation of a human prostate carcinoma cell line (DU 145), Int. J. Cancer 21: 274-281. These cell lines can all be maintained for at least 24 hours in serum-free conditions; thus they are ideal for use in serum-free invasion assays on SU-ECM.

Adult *Strongylocentrotus purpuratus* sea urchins were obtained from Pacific BioMarine, and their embryos were cultured to the early pluteus stage in artificial sea water at 15°C. SU-ECM were prepared from them by treatment with nonionic detergent and strerilized by dilution in the appropriate media.

Cells were harvested by rinsing in Hanks' balanced salt solution, followed by brief treatment with 0.25% trypsin, 0.02% EDTA, and pelleting and resuspension in the appropriate medium with or without 5% FCS at a density of about 50,000 cells per ml. When appropriate, purified bovine plasma fibronectin (Sigma), purified 120 kDa chymotryptic fragment (Gibco BRL). PHSRN or PHSCN peptides (synthesized at the Biomedical Research Core Facilities of the University of Michigan), or GRGDSP or GRGESP peptides (Gibco BRL) were added to the resuspended cells prior to placement of the cells on SU-ECM. In each well of a plate used for an invasion assay, SU-ECM were placed in 0.5 ml of the appropriate medium, and 0.5ml of the resuspended cells dropped on their exterior surfaces. Invasion assays were incubated 1 to 16 hours prior to assay. If some circumstances, invasion assays were fixed in phosphate-buffered saline (PBS) with 2% formaldehyde for 5 minutes at room temperature, then rinsed into PBS.

Invasion assays were coded and scored blindly by microscopic examination under phase contrast at 200- and 400-fold magnification. Each cell contacting an SU-ECM was scored for its position relative to the exterior or interior surfaces. A cell was judged to have invaded if it was located on an interior surface below the focal plane passing through the upper surface of the SU-ECM, but above the focal plane passing through its lower surface. The minimum viability of the cells in each assay was always ascertained at the time of assay by determining the fraction of spread, adherent cells on the bottom of each well scored.

An invasion frequency is defined as the fraction of cells in contact with basement membranes which were located in their interiors at the time of assay. Thus, an invasion frequency of 1 denotes invasion by 100% of the cells in contact with basement membranes. Invasion frequencies were determined multiple times for each cell type assayed. For each type of cell assayed the mean and standard deviation of the invasion frequencies were calculated.

The invasion-inducing sequences of plasma fibronectin were mapped to a peptide sequence 5 amino acids long, the PHSRN peptide, for both metastatic breast and prostate cancer cells. Since the PHSRN sequence is present in plasma fibronectin, a significant component of serum, eliciting the regulatory role of this sequence was only possible because of the availability of a serum-free *in vitro* invasion substrate. It should be noted that neonatal, human fibroblasts are also induced with the PHSRN peptide to invade serum-free SU-ECM. Although fibroblasts do not invade SU-ECM in the presence of serum, the 120 kDa fragment of plasma fibronectin containing the PHSRN sequence can induce fibroblast invasion equally well in the presence of serum or in its absence.

When taken together, the results of experiments showing that the PHSRN sequence of plasma fibronectin induces the invasive behaviors of both metastatic breast and prostate cancer cells, as well as that of normal fibroblasts suggest the intriguing possibility that the invasive behavior associated with tumor cell metastasis may result from defects in the regulation of the normal invasive behaviors associated with wound healing.

### EXAMPLE 6

### Improving Gelatin Depletion As Measured By Fibroblast Invasiveness

In this example, normal, neonatal fibroblasts were tested on the depleted Matrigel material prepared according to Example 3 above (*i*.*e*., antibody depletion). As shown in Figure 4, panning with an antibody after gelatin depletion improved the method for removal, as measured by the reduced invasiveness of fibroblasts. On the other hand, invasiveness of the fibroblasts could be induced by the addition of the PHSRN peptide.

The success of antibody panning suggests the feasibility of removing other components by the antibody panning methods. Other serum components, such as thrombospondin, growth factors and cytokines are contemplated by the present invention for removal by the appropriate (commercially available) antibody.

### EXAMPLE 8

### Inhibiting Invasion Of Human Breast Cancer Cells

In this example, the role of the PHSCN peptide in inhibiting the invasive behavior of metastatic breast cancer cells is demonstrated. The method of Example 4 is employed, with the addition of varying concentrations of the PHSCN peptide.

Example 4 indicates that SUM-52 cells (in medium with 5% fecal calf serum) are induced to invade the SU-ECM substrate in the presence of serum fibronectin or just the PHSRN sequence of fibronectin. Thus, the procedure in Example 4 provides a method for determining the inhibitory potential of the PHSCN peptide by comparing the number of cell invasions in the presence of the PHSCN peptide, with the number of cell invasions in the absence of the PHSCN peptide.

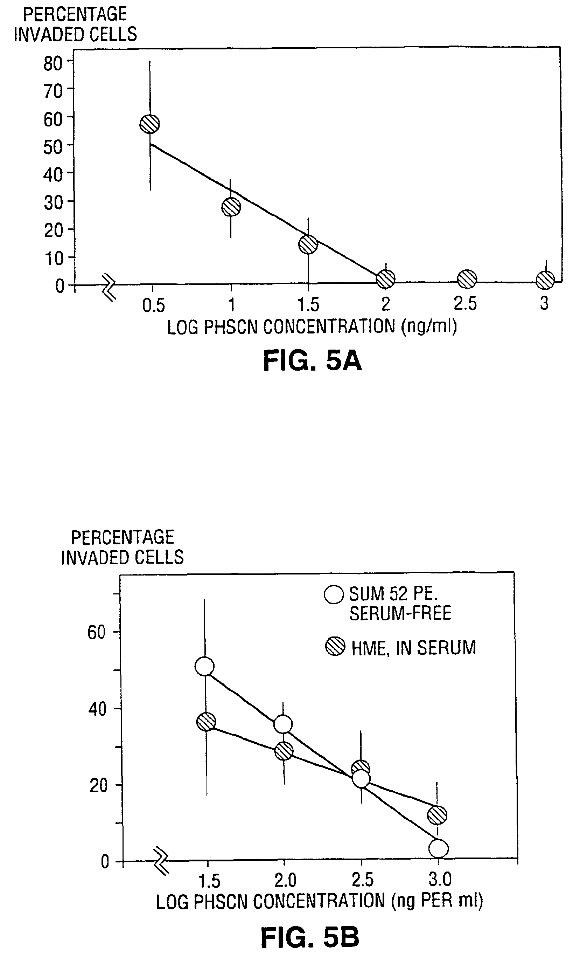

The results of adding varying concentrations of the PHSCN peptide to serum-induced metastatic SUM-52 PE breast cancer cells are presented in Figure 5A. The logs of the PHSCN peptide concentrations in ng per ml are plotted on the X axis. The percentages of invaded SUM 52 PE cells relative to the percentage invaded in the absence of the PHSCN peptide are plotted on the Y axis. Mean invasion percentages are shown with their first standard deviations. Clearly, the PHSCN peptide exhibits a substantial inhibitory affect on these cells, even at relatively low concentrations. The PHSCN peptide's inhibitory affect is further demonstrated by the fact that relatively high concentrations cause complete inhibition.

The results of adding varying concentrations of the PHSCN peptide to PHSRN-induced invasion of both metastatic SUM-52 PE breast cancer cells (in serum free media) and normal human mammary epithelial cells (in 10% FCS), are presented in Figure 5B. All invasion assays were carried out in 100 ng per ml of the PHSRN peptide to induce invasion. Again, the PHSCN peptide exhibits a substantial inhibitory affect on both cell lines at low concentrations, and almost complete inhibition at higher concentrations.

This example demonstrates the PHSCN peptide is an effective inhibitor of human breast cancer cell invasion. In this manner, the PHSCN peptide, or related sequences, are likely to provide effective therapy for human breast cancer by preventing the lethal affects of tumor cell metastasis.

### EXAMPLE 9

### Inhibiting Invasion Of Human Prostate Cancer Cells

In this example, the role of the PHSCN peptide in inhibiting the invasive behavior of metastatic prostate cancer cells is demonstrated. The method of Example 4 is employed, with the addition of varying concentrations of the PHSCN peptide.

Example 4 indicates that DU-145 cells are induced to invade the SU-ECM substrate in the presence of serum fibronectin or just the PHSRN sequence of fibronectin. Thus, the procedure in Example 4 provides a method for determining the inhibitory potential of the PHSCN peptide by comparing the number of cell invasions in the presence of the PHSCN peptide, with the number of cell invasions in the absence of the PHSCN peptide.

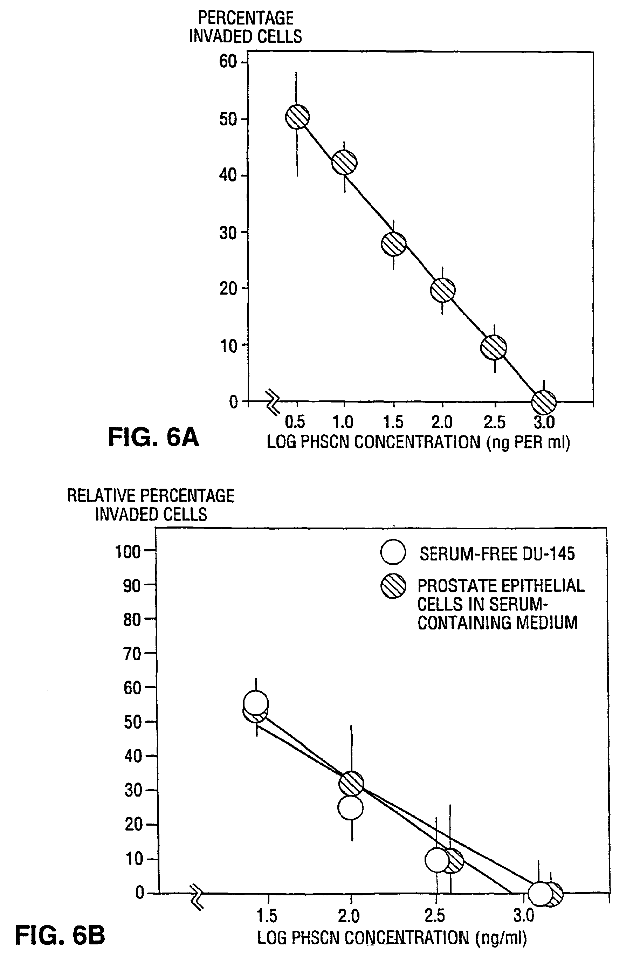

The results of adding varying concentrations of the PHSCN peptide to serum-induced invasion of metastatic DU-145 prostate cancer cells (in 10% serum) are presented in Figure 6A. The logs of the PHSCN concentrations are plotted on the X axis. The percentages of invaded DU-145 cells relative to the percentage invaded in the absence of the PHSCN peptide are plotted on the Y axis. Mean invasion percentages are shown with their first standard deviations. Clearly, the PHSCN peptide exhibits a substantial inhibitory affect on these cells, even at relatively low concentrations. The PHSCN peptide's inhibitory affect is further demonstrated by the fact that relatively high concentrations cause complete inhibition.

The results of adding varying concentrations of the PHSCN peptide to PHSRN-induced metastatic DU-145 prostate cancer cells (in serum-free medium) or to normal human prostate epithelial cells (in 10% FCS), are presented in Figure 6B. All invasion assays were carried out in 100 ng per ml of the PHSRN peptide to induce invasion. Again, the results show that the PHSCN peptide exhibits a substantial inhibitory affect on both cell lines at low concentrations, and almost complete inhibition at higher concentrations.

This example demonstrates the PHSCN peptide is an effective inhibitor of human prostate cancer cell invasion. In this manner, the PHSCN peptide may provide an effective therapy for human prostate cancer by preventing the lethal affects of tumor cell metastasis.

### EXAMPLE 10

### Inhibiting Invasion Of Rat Prostate Cancer Cells

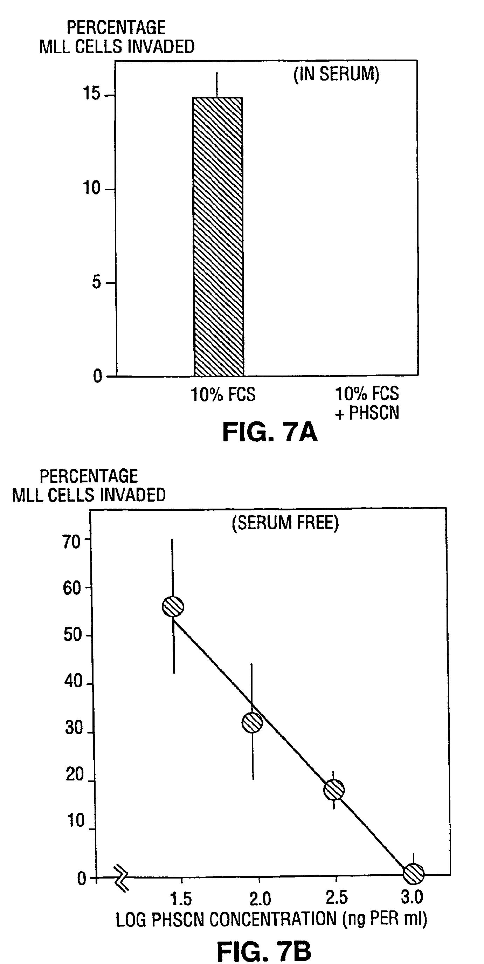

In this example, the role of the PHSCN peptide in inhibiting the invasive behavior of rat metastatic prostate carcinoma MatLyLu (MLL) cells is demonstrated (see Example 4 for the general procedure employed). The result of adding 1 microgram per ml of the PHSCN peptide to serum-induced MLL cells causes complete inhibition of invasion (see Figure 7A).

The result of adding a varying concentration of the PHSCN peptide to PHSRN-induced MLL cells in serum free media is shown in Figure 7B, where 100 ng per ml of PHSRN was used to induce invasion. Figure 7B indicates that the PHSCN peptide exhibits a substantial inhibitory affect even at low concentrations, and almost complete inhibition at higher concentrations. This example demonstrates invasion of rat prostate cancer cells is inhibited in the same manner as human breast cancer cells (see Example 8) and human prostate cancer cells (see Example 9).

### EXAMPLE 11

### Inhibiting Invasion Of Rat Prostate Cancer Cells

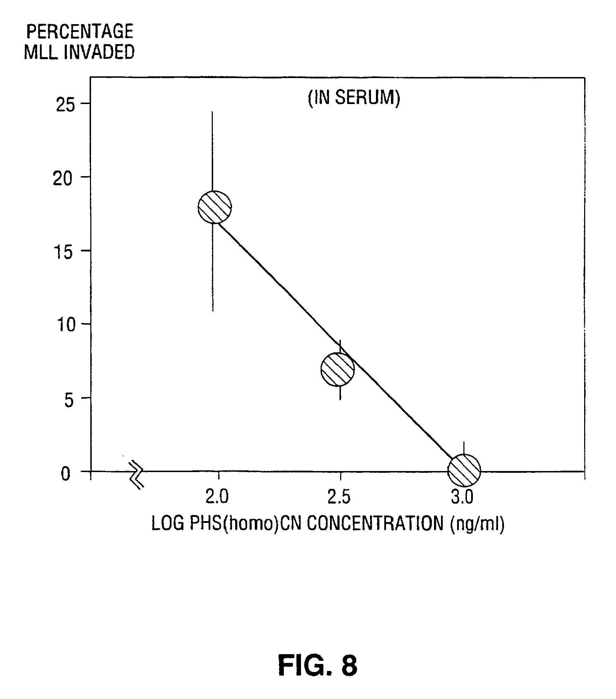

In this example, the role of a homo-cysteine containing peptide (*i*.*e*., PHS(hC)N) in inhibiting the invasive behavior of rat metastatic prostate carcinoma MatLyLu (MLL) cells is demonstrated. The procedure described in Example 10, was employed using SU-ECM substrates in 10% FCS and PHS(hC)N instead of PHSCN. The result of adding varying concentrations of the PHS(hC)N peptide to serum-induced MLL cells indicates this peptide also has an inhibitory affect on cell invasion (see Figure 8). As with the PHSCN peptide, the PHS(hC)N peptide substantially inhibits invasion at lower concentrations, and completely inhibits invasion at higher concentrations. This example demonstrates that the PHS(hC)N peptide has a similar inhibitory affect as the PHSCN peptide. Example 12 describes the use of the peptides for the manufacture of a medicament for treating cancer.

### EXAMPLE 12

### Inhibiting Growth And Metastasis Of Prostate Cancer Tumors

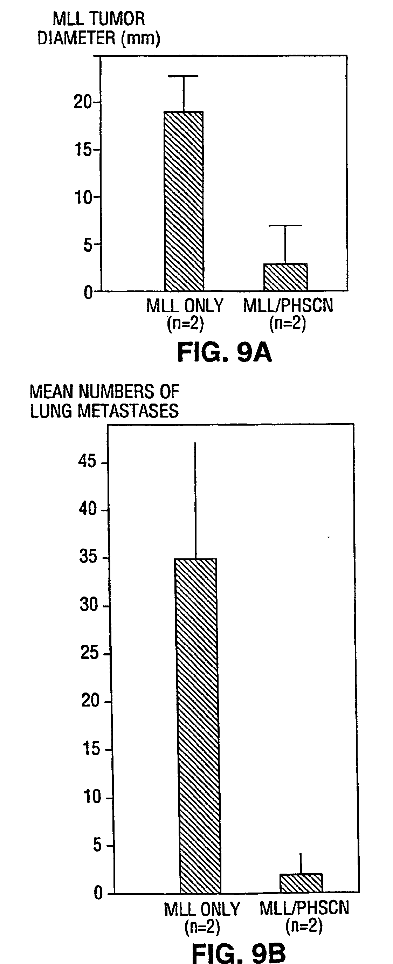

In this example, the role of the PHSCN peptide in inhibiting the growth and metastasis of prostate cancer tumors is demonstrated. In the first part of this example, four Copenhagen rats were injected with 500.000 MatLyLu (MLL) cells subcutaneously in the thigh. Two of these rats also received I mg of the PHSCN peptide along with the injected MLL cells, and thereafter received 1 mg of the PHSCN peptide injected in their tail vein three time per week for two weeks. The other two injected rats were left untreated. Tumor sizes were measured with calipers on day 14, and the tumors in the untreated rats were removed. The results depicted in Figure 9A, clearly demonstrate that the PHSCN peptide significantly slows the growth of injected MLL tumors in rats. It is possible that the ability of the PHSCN peptide to slow tumor growth is due to its inhibition of tumor invasion by normal endothelial cells, an anti-angiogenic effect.

Two weeks after the size of the tumors were measured, the rats were sacrificed and the mean number of lung metastases was determined at 10-fold magnification. The mean number of lung metastases in the untreated mice (MLL only) was nearly 35 in spite of the fact that the initial prostate tumors had been removed when their size was measured. The mean number of lung metastases in the treated mice (MLL + PHSCN) was less than 5, even though the initial prostate tumors were never removed because they were too small. This striking difference in mean number of metastases, depicted in Figure 9B, indicates that the PHSCN peptide significantly inhibits tumor cell metastasis in rats. In this manner, the PHSCN peptide provides effective *in vivo* therapy for cancer by preventing the lethal effects of tumor cell growth and metastasis. Methods of treatment of the human or animal body are not part of the claims. Example 13 describes the use of the peptides for the manufacture of a medicament for treating cancer.

### EXAMPLE 13

### Inhibiting Growth And Metastasis Of Prostate Cancer

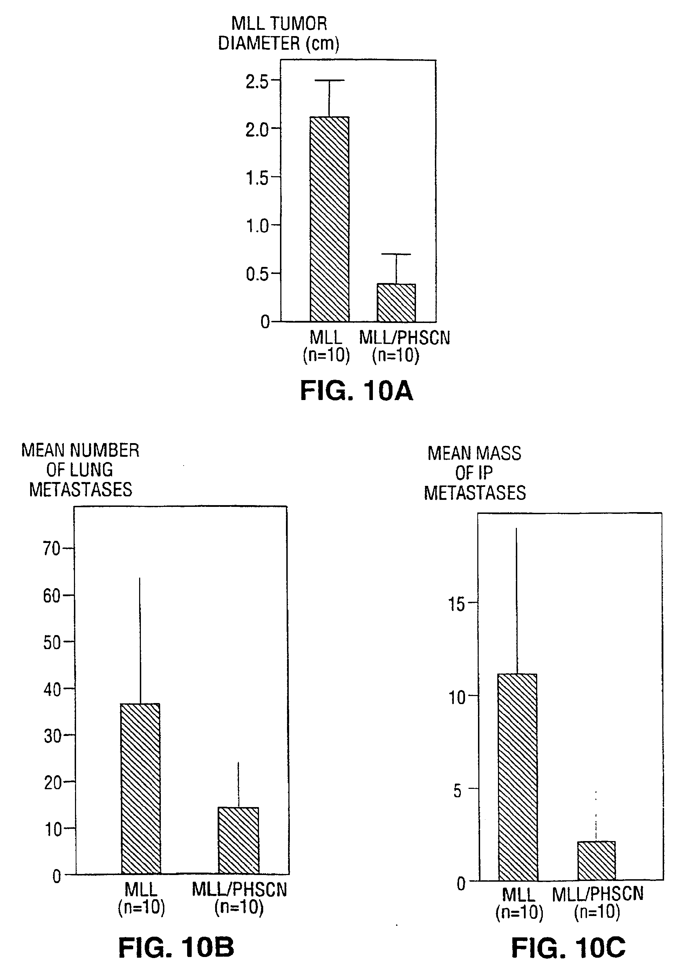

In this example, as in Example 12, the role of the PHSCN peptide in inhibiting the growth and metastasis of prostate cancer tumors *in vivo* is demonstrated. In the first part of this example, 20 Copenhagen rats were injected with 500,000 MatLyuLu (MLL) cells subcutaneously in the thigh. To more closely approximate a real clinical situation. PHSCN peptide treatment of 10 of these rats was initiated after 24 hours, instead of immediately. The 10 treated rats (MLL/PHSCN) received a total of 5 i.v. injections of 1 mg of the PHSCN peptide through the tail vein over two weeks. Tumor sizes were measured with calipers on day 14, and the tumors in the untreated rats were removed. Since the injected tumors in the MLL/PHSCN rats were still small, they were retained in the rats for another 7 to 9 days following the last PHSCN injection. At this time, their sizes were all greater than 2 cm, and they were also removed. The result of the first part of this example, depicted in Figure 10A, clearly indicates that the PHSCN peptide, even when administered after the tumor cells have "seeded", substantially slows the growth of rat prostate cancer tumors.

The dramatic growth-inhibitory effect of the PHSCN peptide on MLL tumors may be due to their inhibition of the invasion of host endothelial cells into the tumor. Host endothelial cell invasion may be induced by the secretion of large amounts of proteinases from the tumors, and the resulting fragmentation of host plasma fibronectin. Fibronectin fragments have been shown to stimulate the migratory/invasive behaviors of normal mesenchymal and endothelial cells. This angiogenic process is believed to occur during normal wound healing. Thus, the ability of metastatic cells to be constitutively induced by intact plasma fibronectin to express proteinases and invade may play a central role both in tumor cell invasion and in tumor growth. In this manner, the PHSCN peptide is an effective chemotherapeutic to prevent the growth of tumors *in vivo*.

In the second part of this example, the MLL/PHSCN rats received 2 more i.v. doses of the PHSCN peptide prior to sacrifice. Ten days after the sizes of the injected primary tumors were determined, all the rats in the two groups (MLL only and MLL/PHSCN) were sacrificed, and the number of lung metastases was determined at 7.5-fold magnification. As can be seen in Figure 10B, there is a significant reduction in the mean numbers of lung metastases in the rats which received PHSCN treatment as compared to the untreated rats.

The 20 rats described in parts one and two of this example were also examined for metastatic tissues in their lymphatic systems. All of these metastases were dissected and weighed. Figure 10C plots the mean masses of intraperitoneal metastases (grams) for the two groups of 10 rats. As is clearly demonstrated, there is a significant reduction in the mean masses of lymphatic metastases in the rats which received PHSCN peptide treatment, as compared to the untreated rats. This may be due to the anti-angiogenic effect of the PHSCN peptide, as described in part one of this example. In this manner, the PHSCN peptide maybe an effective anti-metastatic, growth-inhibiting chemotherapeutic agent for use in the treatment of cancer. Methods of treatment of the human or animal body are not part of the claims.

### EXAMPLE 14

### Chemically Modified Peptides And Cancer Therapy

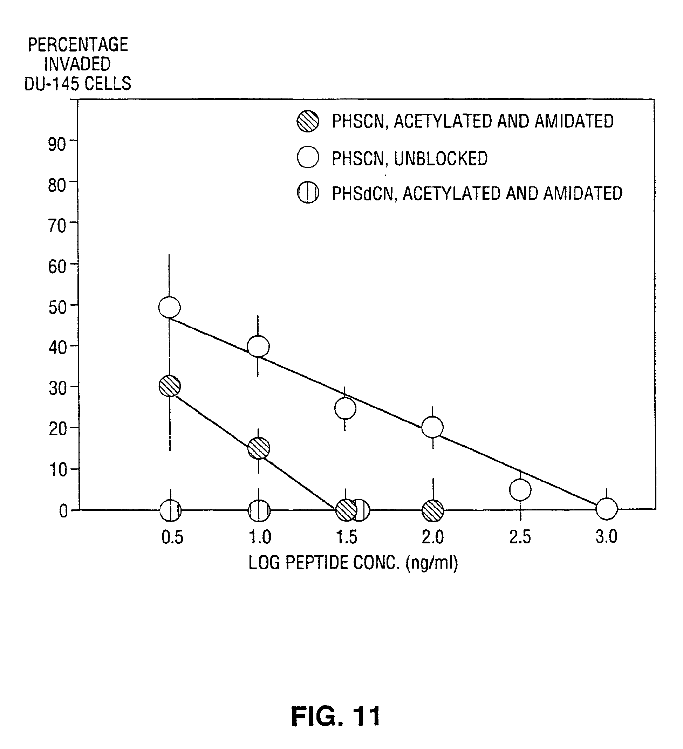

In this example, *in vitro* invasion of cancer cells into substrates is inhibited with peptides that have been chemically modified with protecting groups and peptides having protecting groups as well as the modification wherein an L-amino acid has been replaced with the D-isomer. PHSCN peptide, blocked PHSCN peptide, and blocked PHSCN peptide with a D-cysteine instead of the L-isomer, were tested (Figure 11). The PHSCN peptide without protecting groups (i.e. "unblocked" PHSCN. open circles) completely inhibits the invasion of metastatic DU145 cells in medium with 10% serum at a concentration of 3 ng/ml. When PHSCN is protected from exoproteinase degradation by N-terminal acetylation and C-terminal amidation, the Ac-PHSCN-NH₂ peptide (closed circles) can completely inhibit the invasion of metastatic DU145 cells in medium with 10% serum at a concentration of approximately 1.3 ng/ml. Substitution of the D-isomer of cysteine (making Ac-PHS(dC)N-NH₂ for protection against endoproteolytic activity, striped circles) further increases the invasion-inhibitory activity of the peptide.

While not intending to limit the invention to any particular mechanism, it is believed that increases in activity may be due to proteinase resistance. Such proteases may be found in the serum or may be produced by the DU145 cells (i.e. both membrane-bound and secreted proteases). If proteinase resistance is the reason for the additional activity, this suggests that such modified peptides will have longer circulating half-lives upon administration to animals, including humans. This will permit lower dosages for therapy.

Alternatively, these changes may make the inhibitors interact with the PHSRN-binding pocket of the α5β1 receptor more effectively. That is to say, the addition of groups on the ends of the peptide may have a positioning or steric effect that is beneficial for binding.

### EXAMPLE 15

### Structure/Function Relationships And Designing Non-peptide Mimetics

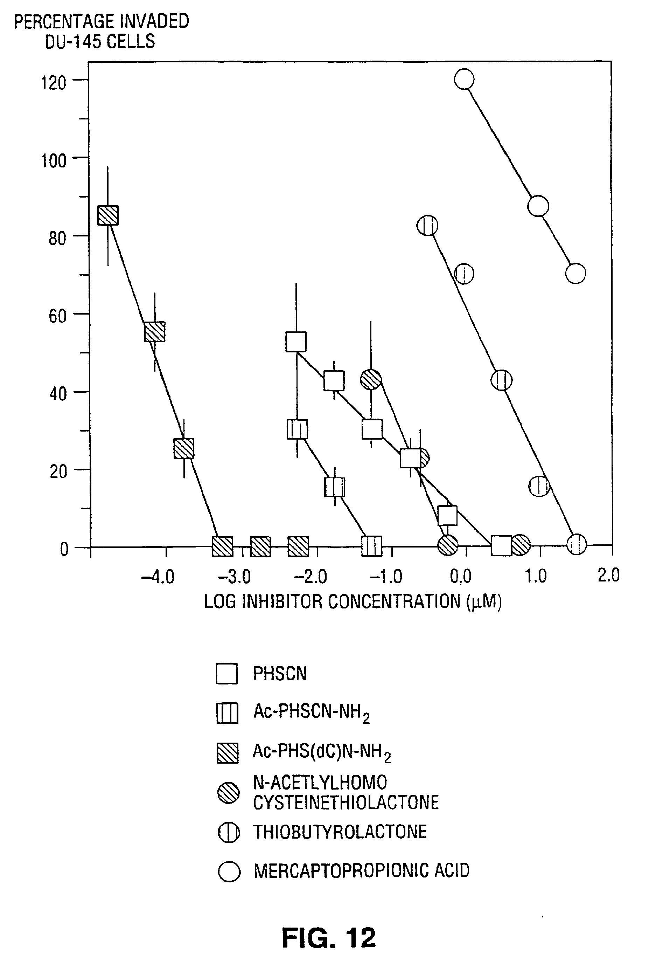

In this example, *in vitro* invasion of cancer cells into substrates is inhibited with non-peptide mimetics in comparison with chemically modified peptides (on a molar basis). The peptides of Example 14 (*i*.*e*. peptides chemically modified with protecting groups and peptides having protecting groups as well as the modification wherein an L-amino acid has been replaced with the D-isomer) were compared with the non-peptide mimetics DL-N-Acetylhomocysteinethiolactone, Thiobutyrolactone, and Mercaptopropionic acid, in order to investigate the structure activity relationship for invasion inhibition by interaction with the PHSRN-binding pocket of the α5β1 fibronectin receptor (Figure 12)

The PHSCN peptide without protecting groups (*i*.*e*. "unblocked" PHSCN, open squares) completely inhibits the invasion of metastatic DU145 cells in medium with 10% serum at a concentration of approximately 2 µM. When PHSCN is protected from exoproteinase degradation by N-terminal acetylation and C-terminal amidation, the Ac-PHSCN-NH₂ peptide (striped squares) can completely inhibit the invasion of metastatic DU145 cells in medium with 10% serum at a concentration of approximately 0.06 µM. Substitution of the D-isomer of cysteine (making Ac-PHS(dC)N-NH₂ for protection against endoproteolytic activity, closed squares) further increases very substantially the invasion-inhibitory activity of the peptide.

The non-peptide mimetic, DL-N-Acetylhomocysteinethiolactone (closed circles) fully inhibits DU145 invasion at a concentration of 0.6 µM. Thus, is about 3-fold more active than the unblocked PHSCN peptide and about 10-fold less active than blocked PHSCN. Thiobutyrolactone (striped circles) fully inhibits DU145 invasion at a concentration of 30 µM. Thus, it is 15 times less active than the unblocked PHSCN peptide and about 500-fold less active than blocked PHSCN. It is also 50-fold less active than N-acetylhomocysteinethiolactone. Mercaptopropionic acid (open circls) did not reduce DU145 invasion by more than 20% at any of the concentrations tested. Thus, it is at least 100 times less active in invasion inhibition than thiobutyrolactone, and 1500 times less active than the unblocked PHSCN peptide.

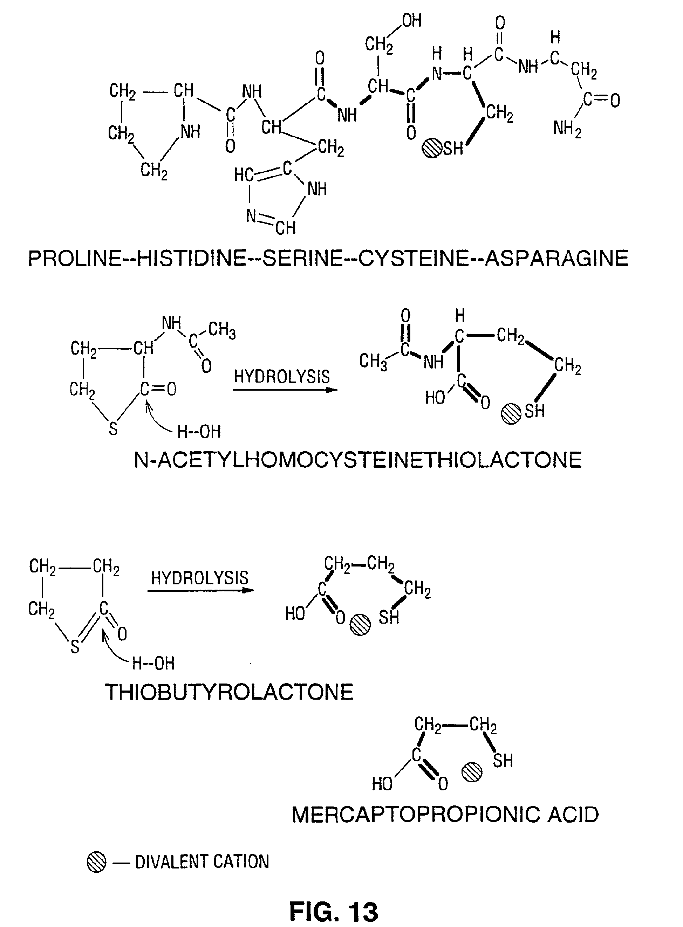

The results of (Figure 12) can be examined in the context of shared structural motifs by comparing the compounds (Figure 13). The PHSCN peptide contains cycsteine and the sulfhydryl group of its cysteine has the potential to form a 6-membered ring by interacting with the carbonyl oxygen of its serine residue to chelate a divalent cation known to reside in the PHSRN-binding pocket of the α5β1 integrin fibronectin receptor. The chelation of this divalent cation may prevents its displacement by the arginine of the PHSRN sequence to activate invasion. This activity may explain how PHSCN functions as a competitive inhibitor of PHSRN-induced invasion. The results with the non-peptide mimetic DL-N-Acetylhomocysteinethiolactone suggest that the amino acids surrounding the cysteine in PHSCN may contribute a 10-fold increase in its invasion-inhibiting activity, presumably by increasing its association with the PHSRN-binding pocket of the α5β1 fibronectin receptor. N-Acetylhomocysteinethiolactone has the possibility of forming a 6-membered ring, very similar to that proposed for the PHSCN invasion-inhibitory peptide, by chelating a divalent cation between its electron-rich sulfhydryl group and its carbonyl oxygen or hydroxyl group (after hydrolysis). It also has attached a nitrogen and a carbonyl group joined by a peptide linkage, which might resemble the peptide bond between the serine and histidine in the PHSCN peptide. These groups may contribute significantly to its invasion-inhibitory activity.

Thiobutyrolactone, like the homocysteinethiolactone described above, can potentially form a 6-membered ring by chelating a divalent cation between its electron-rich sulfhydryl group and its carbonyl oxygen or hydroxyl group (after hydrolysis). However, it lacks the attached peptide-linked nitrogen and carbonyl groups of the thiolactone. Thus, these attached groups may contribute a 50-fold increase in invasion inhibition.

Finally, Mercaptopropionic acid, unlike thiobutyrolactone, has the potential to form only a 5-membered ring upon interaction with the divalent cation. The poor results with this compound suggest that the presence of a 6-membered ring in the inhibitor after divalent cation chelation may increase invasion-inhibitory activity by 100-fold by stabilizing the presence of the divalent cation in the PHSRN-binding pocket. It is known that the divalent cation in this pocket is bound by 4 aspartic acids in the α5 chain of α5β1. It is interesting that the inclusion of this metal ion between the electron-rich -SH and -O or -OH groups would lead to its chelation by 6 electron-rich moities instead of 4. a much more stable configuration. The ability of the PHSCN peptide, N-Acetylhomocysteinethiolactone, and thiobutyrolactone inhibitors to form 6-membered rings as they include the divalent cation in these 6 electron-rich motifs may contribute significantly to their invasion-inhibitory activities because 6-membered organic rings are energetically favored.

The presence of an NH-COOH linked to the 6-membered ring may increase invasion-inhibitory activity by another 50-fold by mimicing the peptide linkage of the serine and histidine. This suggests that this linkage interacts with the amino acids of the PHSRN-binding pocket in a significant way. The presence of the other amino acids in the PHSCN peptide may contribute another 10-fold increase in invasion-inhibitory activity by interacting with the amino acids of the PHSRN-binding pocket of the α5β1 integrin fibronectin receptor.

## Claims

1. An invasion-inhibiting peptide comprising the amino acid sequence X₁HSX₂N, wherein X₁ is proline, and X₂ is an amino acid selected from the group consisting of the L-isomer of cysteine, the D-isomer of cysteine, and homo-cysteine.

2. The invasion-inhibiting peptide of Claim 1, wherein said invasion-inhibiting peptide comprises the amino acid sequence PHSCN.

3. The invasion-inhibiting peptide of claim 1 or 2, wherein said invasion-inhibiting peptide contains additional amino acids added to the amino terminus, the carboxyl terminus, or both the amino and carboxyl termini.

4. The invasion-inhibiting peptide of claim 3, wherein said invasion-inhibiting peptide is between six amino acids and one hundred amino acids in length.

5. The invasion-inhibiting peptide of claim 1, 2 or 3 wherein said invasion-inhibiting peptide's amino terminus is blocked with a protecting group.

6. The invasion-inhibiting peptide of claim 5, wherein said invasion-inhibiting peptide's carboxyl terminus is blocked with a protecting group.

7. The invasion-inhibiting peptide of claim 5, wherein said invasion-inhibiting peptide's amino terminus is blocked with an acetyl group, and said peptide's carboxyl terminus is blocked with an amide group.

8. The invasion-inhibiting peptide of claim 1 or 2, wherein said invasion-inhibiting peptide is resistant to exoproteinases.

9. The invasion-inhibiting peptide of claim 1 or 2, wherein said invasion-inhibiting peptide is resistant to endoproteinases.

10. The invasion-inhibiting peptide of any one of claims 1 to 9, wherein said invasion-inhibiting peptide is suspended in serum-free media.

11. A pharmaceutical formulation comprising the invasion-inhibiting peptide of any one of claims 1 to 10, together with a pharmaceutically acceptable carrier, adjuvant and/or diluent.

12. Use of the pharmaceutical formulation of claim 11 for the manufacture of a medicament for the treatment of cancer.

13. The use of claim 12, wherein said formulation is for administration before or after the surgical removal of a tumor.

14. The use of claim 12, wherein said formulation is for intravenous administration.

15. The formulation of claim 11 for use in treating cancer.

16. The formulation of claim 15, wherein said formulation is for administration before or after the surgical removal of a tumor.

17. The formulation of claim 15, wherein said formulation is for intravenous administration.

18. Use of the invasion-inhibiting peptide of any one of claims 1 to 10 for the preparation of a medicament for the treatment of cancer.

19. The use according to claim 18, wherein said medicament is for administration before or after the surgical removal of a tumor.

20. The use according to claim 18 or 19, wherein said medicament is for intravenous administration.

21. The invasion-inhibiting peptide of any one of claims 1 to 10 for use in treating cancer.

22. The invasion-inhibiting peptide of claim 21 for administration before or after the surgical removal of a tumor.

23. The invasion-inhibiting peptide of claim 21 or 22 for intravenous administration.

## Patentansprüche

1. Invasions-inhibierendes Peptid umfassend die Aminosäuresequenz X₁HSX₂N, worin X₁ Prolin ist, und X₂ ist eine Aminosäure ausgewählt aus der Gruppe bestehend aus dem L-Isomer von Cystein, dem D-Isomer von Cystein und Homo-Cystein.

2. Invasions-inhibierendes Peptid nach Anspruch 1, worin das Invasions-inhibierende Peptid die Aminosäuresequenz PHSCN umfasst.

3. Invasions-inhibierendes Peptid nach Anspruch 1 oder 2, worin das Invasions-inhibierende Peptid zusätzlich an dem Aminoterminus, an dem Carboxylterminus, oder an beiden von Amino- und Carboxylterminus hinzugefügte Aminosäuren enthält.

4. Invasions-inhibierendes Peptid nach Anspruch 3, worin das Invasions-inhibierende Peptid von einer Länge zwischen sechs Aminosäuren und einhundert Aminosäuren ist.

5. Invasions-inhibierendes Peptid nach Anspruch 1, 2 oder 3, worin der Aminoterminus des Invasions-inhibierenden Peptids mit einer schützenden Gruppe blockiert ist.

6. Invasions-inhibierendes Peptid nach Anspruch 5, worin der Carboxylterminus des Invasions-inhibierenden Peptids mit einer schützenden Gruppe blockiert ist.

7. Invasions-inhibierendes Peptid nach Anspruch 5, worin der Aminoterminus des Invasions-inhibierenden Peptids mit einer Azetylgruppe blockiert ist, und der Carboxylterminus des Peptids mit einer Amidgruppe blockiert ist.

8. Invasions-inhibierendes Peptid nach Anspruch 1 oder 2, worin das Invasions-inhibierende Peptid resistent gegen Exoproteinasen ist.

9. Invasions-inhibierendes Peptid nach Anspruch 1 oder 2, worin das Invasions-inhibierende Peptid resistent gegen Endoproteinasen ist.

10. Invasions-inhibierendes Peptid nach jedem der Ansprüche 1 bis 9, worin das Invasions-inhibierende Peptid in einem Serum-freien Medium suspendiert ist.

11. Pharmazeutische Formulierung umfassend das Invasions-inhibierende Peptid nach einem der Ansprüche 1 bis 10, zusammen mit einem pharmazeutisch akzeptablen Träger, Adjuvanz und/oder Verdünnungsmittel.

12. Verwendung der pharmazeutischen Formulierung nach Anspruch 11 für die Herstellung eines Medikaments zur Behandlung von Krebs.

13. Verwendung nach Anspruch 12, worin die Formulierung für die Administration vor oder nach dem chirurgischen Entfernen eines Tumors ist.

14. Verwendung nach Anspruch 12, worin die Formulierung für die intravenöse Administration ist.

15. Formulierung nach Anspruch 11 zur Verwendung in der Behandlung von Krebs.

16. Formulierung nach Anspruch 15, worin die Formulierung für die Administration vor oder nach dem chirurgischen Entfernen eines Tumors ist.

17. Formulierung nach Anspruch 15, worin die Formulierung für die intravenöse Administration ist.

18. Verwendung des Invasions-inhibierenden Peptides nach einem der Ansprüche 1 bis 10 für die Herstellung eines Medikaments zur Behandlung von Krebs.

19. Verwendung nach Anspruch 18, worin das Medikament für die Administration vor oder nach dem chirurgischen Entfernen eines Tumors ist.

20. Verwendung nach Anspruch 18 oder 19, worin das Medikament für die intravenöse Administration ist.

21. Invasions-inhibierendes Peptid nach einem der Ansprüche 1 bis 10 zur Verwendung in der Behandlung von Krebs.

22. Invasions-inhibierendes Peptid nach Anspruch 21 für die Administration vor oder nach dem chirurgischen Entfernen eines Tumors.

23. Invasions-inhibierendes Peptid nach Anspruch 21 oder 22 für die intravenöse Administration.

## Revendications

1. Peptide anti-invasif comprenant la séquence d'acides aminés X₁HSX₂N, où X₁ est la proline et X₂ est un acide aminé choisi dans le groupe constitué par l'isomère L de la cystéine, l'isomère D de la cystéine et l'homocystéine.

2. Peptide anti-invasif selon la revendication 1, dans lequel ledit peptide anti-invasif comprend la séquence d'acides aminés PHSCN.

3. Peptide anti-invasif selon la revendication 1 ou 2, dans lequel ledit peptide anti-invasif contient des acides aminés supplémentaires ajoutés à la terminaison amino, la terminaison carboxyle ou les terminaisons amino et carboxyle.

4. Peptide anti-invasif selon la revendication 3, dans lequel ledit peptide anti-invasif a une longueur comprise entre six acides aminés et cent acides aminés.

5. Peptide anti-invasif selon la revendication 1, 2 ou 3, dans lequel la terminaison amino dudit peptide anti-invasif est bloquée avec un groupe protecteur.

6. Peptide anti-invasif selon la revendication 5, dans lequel la terminaison carboxyle dudit peptide anti-invasif est bloquée par un groupe protecteur.

7. Peptide anti-invasif selon la revendication 5, dans lequel la terminaison amino dudit peptide anti-invasif est bloquée par un groupe acétyle, et la terminaison carboxyle dudit peptide est bloquée par un groupe amide.

8. Peptide anti-invasif selon la revendication 1 ou 2, dans lequel ledit peptide anti-invasif est résistant aux exoprotéinases.

9. Peptide anti-invasif selon la revendication 1 ou 2, dans lequel ledit peptide anti-invasif est résistant aux endoprotéinases.

10. Peptide anti-invasif selon l'une quelconque des revendications 1 à 9, dans lequel ledit peptide anti-invasif est mis en suspension dans des milieux sans sérum.

11. Formulation pharmaceutique comprenant le peptide anti-invasif selon l'une quelconque des revendications 1 à 10, ainsi qu'un excipient, un adjuvant et/ou un diluant pharmaceutiquement acceptables.

12. Utilisation de la formulation pharmaceutique selon la revendication 11 pour la fabrication d'un médicament pour le traitement du cancer.

13. Utilisation selon la revendication 12, dans laquelle ladite formulation est destinée à une administration avant ou après l'ablation chirurgicale d'une tumeur.

14. Utilisation selon la revendication 12, dans laquelle ladite formulation est destinée à une administration par voie intraveineuse.

15. Formulation selon la revendication 11, destinée à une utilisation dans le traitement du cancer.

16. Formulation selon la revendication 15, dans laquelle ladite formulation est destinée à une administration avant ou après l'ablation chirurgicale d'une tumeur.

17. Formulation selon la revendication 15, dans laquelle ladite formulation est destinée à une administration par voie intraveineuse.

18. Utilisation du peptide anti-invasif selon l'une quelconque des revendications 1 à 10 pour la préparation d'un médicament pour le traitement du cancer.

19. Utilisation selon la revendication 18, dans laquelle ledit médicament est destiné à une administration avant ou après l'ablation chirurgicale d'une tumeur.

20. Utilisation selon la revendication 18 ou 19, dans laquelle ledit médicament est destiné à une administration par voie intraveineuse.

21. Peptide anti-invasif selon l'une quelconque des revendications 1 à 10 pour une utilisation dans le traitement du cancer.

22. Peptide anti-invasif selon la revendication 21, pour une administration avant ou après l'ablation chirurgicale d'une tumeur.

23. Peptide anti-invasif selon la revendication 21 ou 22, pour une administration par voie intraveineuse.
